(19) Europäisches Patentamt
European Patent Office
Office européen des brevets

(11) **EP 4 049 643 B1**

(12) **EUROPEAN PATENT SPECIFICATION**

(45) Date of publication and mention
of the grant of the patent:
**28.08.2024 Bulletin 2024/35**

(21) Application number: **22158227.3**

(22) Date of filing: **23.02.2022**

(51) International Patent Classification (IPC):
***A61G 7/057*** *(2006.01)*

(52) Cooperative Patent Classification (CPC):
**A61G 7/05769;** A61G 2203/34; A61G 2203/44

(54) **APPARATUS AND METHOD FOR DISCRIMINATING PATIENT MOVEMENT ON A SUPPORT APPARATUS BETWEEN SELF MOVEMENT AND ASSISTED MOVEMENT**

VORRICHTUNG UND VERFAHREN ZUR UNTERSCHEIDUNG ZWISCHEN SELBST- UND FREMDBEWEGUNG EINES PATIENTEN AUF EINER STÜTZVORRICHTUNG

APPAREIL ET PROCÉDÉ PERMETTANT DE DISCRIMINER LE MOUVEMENT D'UN PATIENT SUR UN APPAREIL DE SUPPORT ENTRE UN MOUVEMENT AUTONOME ET UN MOUVEMENT ASSISTÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2021 US 202163154057 P**

(43) Date of publication of application:
**31.08.2022 Bulletin 2022/35**

(73) Proprietor: **Hill-Rom Services, Inc.**
**Batesville, IN 47006-9167 (US)**

(72) Inventor: **RECEVEUR, Timothy J**
**Batesville, 47006-9167 (US)**

(74) Representative: **Reddie & Grose LLP**
**The White Chapel Building**
**10 Whitechapel High Street**
**London E1 8QS (GB)**

(56) References cited:
**US-A1- 2013 145 558      US-A1- 2015 008 049**
**US-A1- 2017 035 360      US-A1- 2020 297 564**

**Description**

The present disclosure relates to the use of sensors of a patient support apparatus, such as a hospital bed, for example, to detect patient motion and characterize the patient motion. More specifically, the present disclosure is directed to combining signals from load cells of a scale system and the pressure sensors of an inflatable mattress of a typical hospital bed to act as an instrument to assess patient motion and use the patient motion to improve the treatment of the patient.

US 2020/0297564 A1 describes a patient support including a plurality of inflatable bladders. Depth sensors are included in the support that measure the degree of penetration of a patient into the mattress. An air pressure sensor is also included that measures the pressure inside at least one bladder. A suitable inflation level of the mattress is determined by monitoring the rate of change of the depth with respect to air pressure as the bladder is either inflated or deflated. By detecting an inflection point in the graphical relationship of the depth and pressure outputs, a suitable inflation point for the bladders is determined that reduces interface pressures experienced by the patient, yet does not overly sink the patient into the mattress to a degree of discomfort.

The use of load cells in patient support apparatuses, such as hospital beds, for example, to measure patient weight is known. Over time, approaches to using the information from the load cells to detect patient movement and to issue an alert or notification when the patient moves beyond a particular threshold have been developed. The use of load cells to make these determinations and inferences based on the motion is limited by the potential for external influences, such as the addition of equipment to the frame supported on the scale. When this is done, the existing information regarding the position of the patient is compromised as the weight distribution is changed unexpectedly.

The pressure sensors used to measure air pressure in zones of an inflatable mattress are used to control the inflation pressure in the zones to control the interface pressure experienced by a patient supported on the mattress. However, because of transient effects and lack of precision, air pressure sensors associated with mattress zones are not regularly used to measure patient information. The challenges of using air pressure sensors are exacerbated by the variability between the anthropometric characteristics of patients. People with completely different body types sometimes have similar weights. The variations in the surface area of bodies can vary the pressure and volume effects applied to inflatable zones. Still further, the variability in the construction of chambers in the zones makes every application necessary to characterize.

In addition, caregivers or visitors may intermittently apply pressure to the mattress, thereby changing air pressure measurements and the distribution of the weight on the frame. Motion algorithms generally rely on changes in the distribution of weight over multiple sensors to determine patient location and relative movement. These transient and external forces confound the algorithms used to determine patient movement and motion.

In some cases, it is important to determine patient movement relative to the patient support apparatus. Movement in this context means a change in position of the patient on the patient support apparatus, such as rolling over or moving toward an edge of a patient support apparatus to exit the patient support apparatus, for example. In other cases, it is important to identify patient motion such as movement of limbs or oscillating motion that does not result in a change of position or movement of the patient relative to the patient support apparatus, but is still useful in predicting patient outcomes or potential future patient movements.

Thus, there is a need to improve the approaches to measuring and characterizing patient motion and movement in real-time. Improving the characterization of patient motion allows for a more fulsome predictive analysis of patient actions and evaluation of patient conditions. For example, as a patient progresses out of sedation, certain motions are indicative of the state of consciousness of the patient. Also, certain motions are indicative of a patient preparing to make a movement such that monitoring for the motion provides an advanced indication that may be used to alert a caregiver that a patient is preparing to make an attempt to exit the patient support apparatus.

The present disclosure includes one or more of the features recited in the appended claims and/or the following features which, alone or in any combination, may comprise patentable subject matter.

The present invention is defined by the appended independent claims.

According to a first aspect of the present disclosure, a patient support apparatus comprises a plurality of load cells, a frame supported on the load cells, a mattress, a plurality of air pressure sensors, and a control system. The mattress includes a plurality of inflatable zones positioned on the frame, the mattress and frame cooperating to direct any patient load through the mattress and frame to the load cells. The each of the plurality of air pressure sensors measures the pressure in a respective inflatable zone of the mattress. The control system includes a controller operable to receive a separate signal from each of the plurality of load cells and each of the plurality of air pressure sensors and process the signals to identify, based on transient changes in the signals, motion of the patient that does not result in relative movement of the patient relative to the frame. The motion of the patient is further processed by the controller to characterize the nature of the patient motion and, based on the characterization of the patient motion, the controller automatically updating a patient profile in a patient record to reflect the characterization of the patient motion.

In some embodiments, the controller is operable to monitor the energy detected by each of the load cells and each of the air pressure sensors and compare the change in total energy measured by the load cells and air pressure sensors to determine if external energy has acted on the patient support apparatus. If a change in total energy measured is indicative that external energy has acted on the patient support apparatus, the controller utilizes the resulting change in total energy to modify the characterization of the patient motion that is used to update the patient profile.

In some embodiments, the controller identifies that the transient changes in the signals are indicative of motion of a least a portion of the patient in a vertical direction. In some embodiments, the controller is operable to calculate the work done by the patient in the vertical direction to characterize the patient motion.

In some embodiments, the controller is operable to compare the air pressure sensor signal for each of two adjacent zones and use the relative changes in the pressure in the adjacent zones during a potential patient movement event to confirm the characterization of the patient motion.

In some embodiments, the potential patient motion is characterized as a self-offloading motion in which a patient readjusts their position on the mattress without any external influence.

In some embodiments, the potential patient motion is characterized as a non-patient movement artifact in which a patient's position is changed on the mattress with external influence.

In some embodiments, the potential patient motion is characterized as a patient lateral motion in which a patient readjusts their position on the mattress without any external influence.

In some embodiments, the controller is operable to determine that the characterization of the patent motion results in an adverse condition for the patient and issues an alert indicative of the adverse condition.

According to a second aspect of the present disclosure, a system comprises a patient support surface including a plurality of inflatable zones, a plurality of load cells supporting the patient support surface, a plurality of air pressure sensors, each pressure sensor measuring the pressure in a respective inflatable zone of the patient support surface, and a controller. The controller is operable to receive a separate signal from each of the plurality of load cells and each of the plurality of air pressure sensors, process the signals to identify motion of the patient. The controller is further operable to process the motion of the patient to characterize the nature of the patient motion and, based on the characterization of the patient motion, automatically update a patient profile in a patient record to reflect the characterization of the patient motion.

In some embodiments, the controller is operable to monitor the energy detected by each of the load cells and each of the air pressure sensors and compare the change in total energy measured by the load cells and air pressure sensors to determine if external energy has acted on the system. If a change in total energy measured is indicative that external energy has acted on the system, the controller utilizes the resulting change in total energy to modify the characterization of the patient motion that is used to update the patient profile.

In some embodiments, the signals are determined to be indicative of motion of a least a portion of the patient in a vertical direction. In some embodiments, the controller is operable to calculate the work done by the patient in the vertical direction to characterize the patient motion.

In some embodiments, the controller is operable to compare the air pressure sensor signal for each of two adjacent zones and use the relative changes in the pressure in the adjacent zones during a potential patient movement event to confirm the characterization of the patient motion.

In some embodiments, the potential patient motion is characterized as a self-offloading motion in which a patient readjusts their position on the patient support surface without any external influence.

In some embodiments, the potential patient motion is characterized as a non-patient movement artifact in which a patient's position is changed on the patient support surface with external influence.

In some embodiments, the potential patient motion is characterized as a patient lateral motion in which a patient readjusts their position on the patient support surface without any external influence.

In some embodiments, the controller is operable to determine that the characterization of the patient motion results in an adverse condition for the patient and issues an alert indicative of the adverse condition.

According to a third aspect of the present disclosure, a method of characterizing motion of a person on an inflatable mattress having multiple inflatable zones, comprises the steps of monitoring signals from a plurality of pressure sensors, each pressure sensor providing a signal indicative of the pressure in a respective inflatable zone, monitoring signals from a plurality of load cells, the plurality of load cells supporting inflatable mattress, processing the signals from the load cells and pressure sensors to identify motion of the person. The method further comprises that upon detection of a potential motion of the person, further processing the signals to characterize the nature of the person's motion. The method still further comprises, based on the characterization of the motion, automatically updating a record associated with the particular person to reflect the characterization of the motion of person.

In some embodiments, the method further includes monitoring the energy detected by each of the load cells and each of the air pressure sensors, comparing the change in total energy measured by the load cells and air pressure sensors to determine if external energy other than the person supported on the mattress has acted on the mattress, and if a change in total energy measured is indicative that external energy has acted on the mattress, utilizing the resulting change in total energy to modify the characterization of the motion that is used to update the record.

In some embodiments, the method further includes determining that the signals are indicative of motion of a least a portion of the person in a vertical direction.

In some embodiments, the method further includes calculating the work done by the person in the vertical direction to characterize the motion.

In some embodiments, the method further includes comparing the air pressure sensor signal for each of two adjacent zones, and using the relative changes in the pressure in the adjacent zones during a potential person movement event to confirm the characterization of the motion.

In some embodiments, the method further includes monitoring the energy detected by each of the load cells and each of the air pressure sensors, comparing the change in total energy measured by the load cells and air pressure sensors to determine if external energy has acted on the mattress, and if a change in total energy measured is indicative that external energy has acted on the mattress, utilizing the resulting change in total energy to modify the characterization of the motion that is used to update the record.

In some embodiments, the method further includes characterizing the potential motion as a self-offloading motion in which a person readjusts their position on the mattress without any external influence.

In some embodiments, the method further includes the potential motion being characterized as a non-person movement artifact in which a person's position is changed on the mattress with external influence.

In some embodiments, the method further includes potential motion being characterized as a lateral motion in which the person readjusts their position on the mattress without any external influence.

In some embodiments, the method further includes determining that the characterization of the motion results in an adverse condition for the person and issues an alert indicative of the adverse condition.

Additional features, which alone or in combination with any other feature(s), such as those listed above and/or those listed in the claims, can comprise patentable subject matter and will become apparent to those skilled in the art upon consideration of the following detailed description of various embodiments exemplifying the best mode of carrying out the embodiments as presently perceived.

The invention will now be further described by way of example with reference to the accompanying drawings, in which:

Fig. 1 is a perspective view of a patient support apparatus including a control system operable to measure signals from a plurality of sensors and process those signals according to the present disclosure;

Fig. 2 is a block diagram of a portion of the control system of the patient support apparatus of Fig. 1;

Fig. 3 is a diagrammatic illustration of the interaction between a first frame of the patient support apparatus of Fig. 1 and a second frame supported on load cells supported from the first frame;

Fig. 4, is a side view of a portion of the patient support apparatus of Fig. 1 showing a first frame supported on load cells supported on a second frame, the load cells supporting all of the load of the first frame;

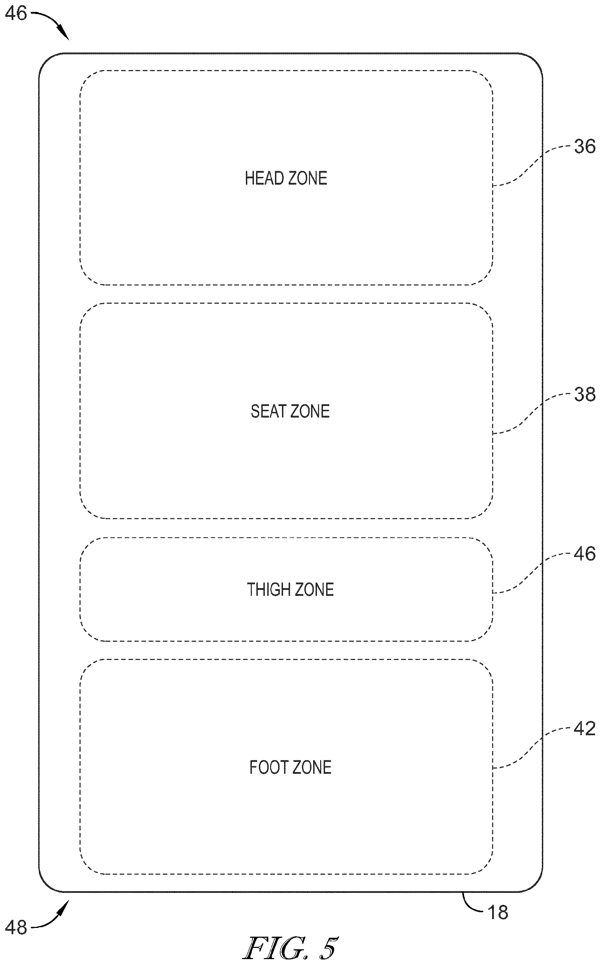

Fig. 5 is a diagrammatic representation of a mattress of the patient support apparatus of Fig. 1, the mattress including multiple inflatable zones;

Fig. 6 is a graphical representation of a group of data collected from the patient support apparatus of Fig. 1, the graphical representation showing the difference between incidents of patient movement detected by the sensors of the patient support apparatus and non -patient movement artifacts detected by the sensors;

Fig. 7 is a diagrammatic representation of an algorithm for characterizing sensor signals from the patient apparatus of Fig 1;

Fig. 8 is a graphical representation of a time series of a sensor signal from a group of sensor of the patient support apparatus of Fig. 1;

Fig. 9 is a view similar to Fig. 4, however, the patient shown in Fig. 9 is in the process of making a self-offloading patient movement; and

Fig. 10 is a view similar to Figs. 4 and 9, but the patient in Fig. 10 has moved to a new position on the mattress of the patient support apparatus.

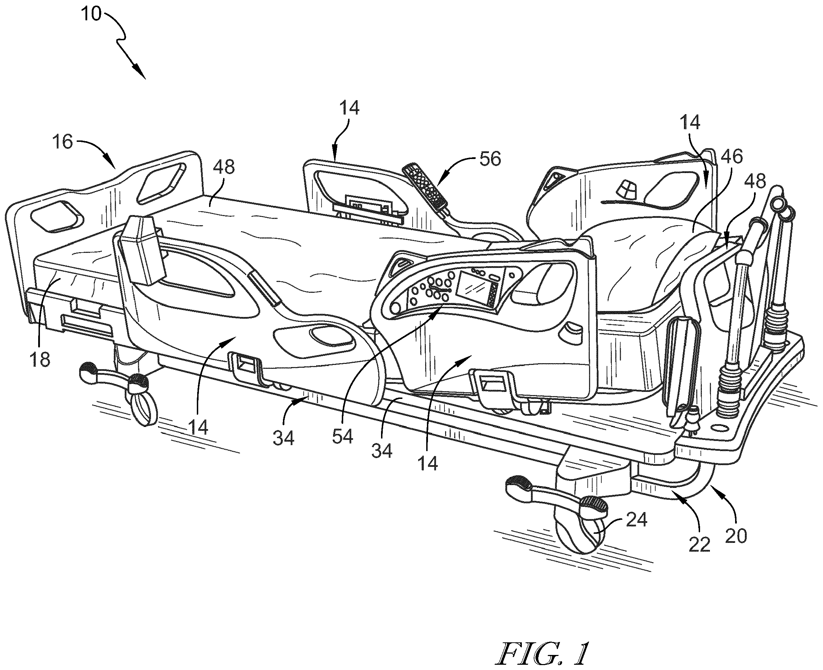

An illustrative patient support apparatus 10 embodied as a hospital bed is shown in Fig. 1. The bed 10 of Fig. 1 has a frame 20 which includes a base frame 22 supported on casters 24. The stationary base frame 22 further supports a weigh frame 30 that an adjustably positionable mattress support upper frame 34 supporting a mattress 18. The illustrative mattress 18 is an inflatable patient support surface which includes inflatable zones including a head zone 36, a seat zone 38, thigh zone 40, and a foot zone 42. The bed 10 further includes a headboard 12 at a head end 46 of the bed 10, a footboard 16 at a foot end 48 of the bed 10, and a movable siderails 14 coupled to the upper frame 34 of the bed 10. The bed 10 also includes a user interface 54 positioned on one of the siderails 14. The bed 10 of the embodiment of Fig. 1 is conventionally configured to adjustably position the upper frame 34 relative to the base frame 22 to adjust the position of a patient supported on the mattress 18.

Conventional structures and devices may be provided to adjustably position the upper frame 34, and such conventional structures and devices may include, for example, linkages, drives, and other movement members and devices coupled between base frame 22 and the weigh frame 30, and/or between weigh frame 30 and upper frame 34. Control of the position of the upper frame 34 and mattress 18 relative to the base frame 22 or weigh frame 30 is controlled, for example, by a patient control pendant 56 or user interface 54. The upper frame 34 may, for example, be adjustably positioned in a general incline from the head end 46 to the foot end 48 or vice versa. Additionally, the upper frame 34 may be adjustably positioned such that the head section 44 of the mattress 18 is positioned between minimum and maximum incline angles, e.g., 0-65 degrees, relative to horizontal or bed flat, and the upper frame 34 may also be adjustably positioned such that a seat section (not shown) of the mattress 18 is positioned between minimum and maximum bend angles, e.g., 0-35 degrees, relative to horizontal or bed flat. Those skilled in the art will recognize that the upper frame 34 or portions thereof may be adjustably positioned in other orientations, and such other orientations are contemplated by this disclosure.

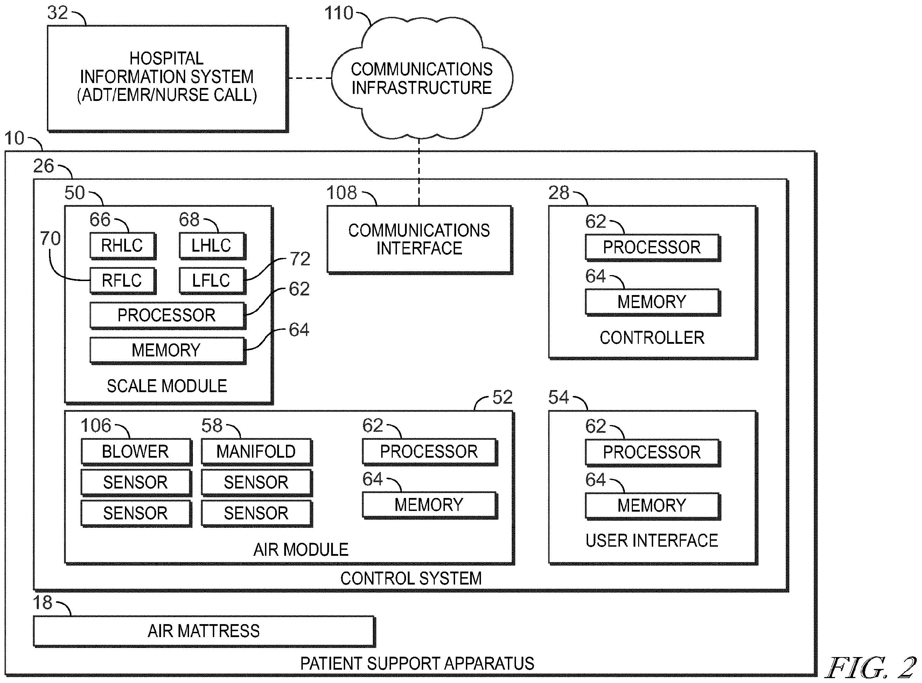

In one illustrative embodiment shown diagrammatically in Fig. 2, the bed 10 has a control system 26 that includes a controller 28, a scale module 50, an air module 52, and the user interface 54. In the illustrative embodiment each of the controller 28, scale module 50, air module 52, and user interface 54 includes a processor 62 and a memory device 64. The processor 62 and memory device 64 are shown only with respect to the controller 28, but similar structures are used in the scale module 50, air module 52, and user interface 54. The memory device 64 includes instructions that, when executed by the processor 62, cause the processor 62 to perform functions as associated with the particular one of controller 28, scale module 50, air module 52, and user interface 54. The components of the control system 26 communicate amongst themselves to share information and distribute the functions of the bed 10. The processor 62 of each of the controller 28, scale module 50, air module 52, and user interface 54 is also operable, based on instructions from the memory device 64, to communicate with the others of the controller 28, scale module 50, air module 52, and user interface 54 using a communications protocol. It should be understood that the term processor here includes any microprocessor, microcontroller, processor circuitry, control circuitry, preprogrammed device, or any structure capable of accessing the memory device and executing non-transient instructions to perform the tasks, algorithm, and processed disclosed herein. In the illustrative embodiment, the control system 26 employs a conventional controller area network (CAN) for communications between subsystems, but it should be understood that any of a number of networking and communications solutions could be employed in the control system 26.

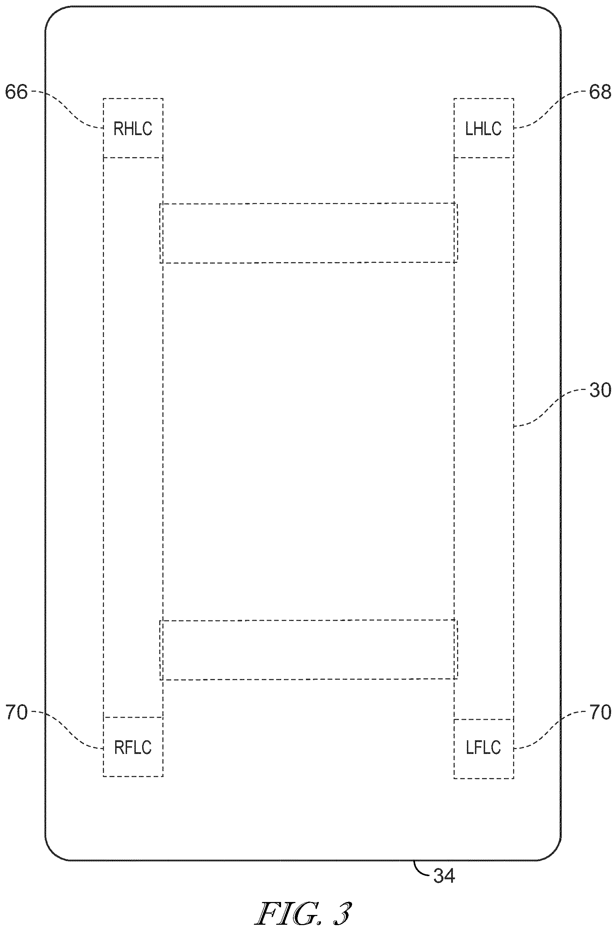

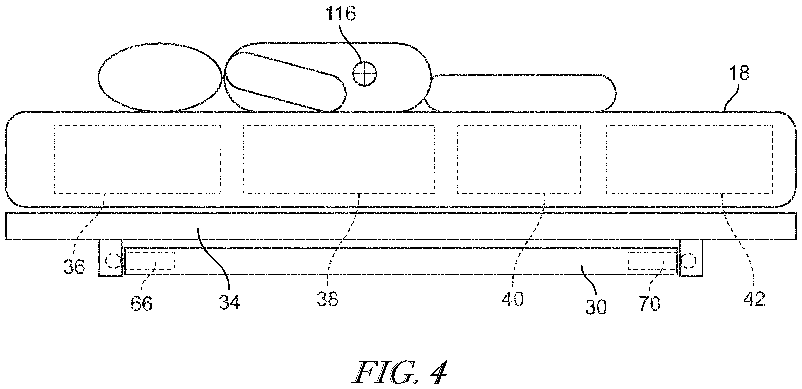

The scale module 50 includes four load cells 66, 68, 70, and 72. To determine a weight of a patient supported on the mattress 18, the load cells 66, 68, 70, and 72 are positioned between the weigh frame 30 and the upper frame 34 as illustrated in Figs. 3 and 4. Each load cell 66, 68, 70, 72 is configured to produce a signal indicative of a load supported by the respective load cell 66, 68, 70, 72 from the upper frame 34 relative to the weigh frame 30. Some of the structural components of the bed 10 will be designated hereinafter as "right", "left", "head" and "foot" from the reference point of an individual lying on the individual's back on the mattress 18 with the individual's head oriented toward the head end 46 of the bed 10 and the individual's feet oriented toward the foot end 48 of the bed 10. Following this convention, the load cell 66 is designated as the right head load cell (RHLC) in the figures to represent that the load cell 66 is positioned at the right side of the bed 10 at the head end 46. The load cell 68 is designated at the left head load cell (LHLC), the load cell 70 is designated as the right foot load cell (RFLC), and the load cell is designated left foot load cell (LFLC), each following the same convention.

The scale module 50 includes a processor 62 that is in communication with each of the respective load cells 66, 68, 70, and 72 and operable to process the signals from the load cells 66, 68, 70, and 72. The memory device 64 is also utilized by the controller 28 to store information corresponding to features and functions provided by the bed 10.

A weight distribution of a load among the plurality of load cells 66, 68, 70, and 72 may not be the same depending on variations in the structure of the bed 10, variations in each of load cells 66, 68, 70, and 72 and the position of the load on the mattress 18 relative to the particular load cell 66, 68, 70, or 72. Accordingly, a calibration constant for each of the load cells 66, 68, 70, and 72 is established to adjust for differences in the load cells 66, 68, 70, and 72 in response to the load borne by each. Each of the load cells 66, 68, 70, and 72 produces a signal indicative of the load supported by that load cell 66, 68, 70, or 72. The loads detected by each of the respective load cells 66, 68, 70, 72 are adjusted using a corresponding calibration constant for the respective load cell 66, 68, 70, 72. The adjusted loads are then combined to establish the actual weight supported on the bed 10. In the present disclosure, the independent signals from each of the load cells 66, 68, 70, 72 is used to draw inferences about the movement and motion of the patient.

The air module 52 is the functional controller for the mattress 18 and includes processor 62 and a memory device 64. The processor 62 is in communication with a blower 106, a manifold 58, and an air pressure sensor assembly 60. The air module 52 is a conventional structure with the manifold 58 operating under the control of the processor 62 to control the flow of air from the blower 106 into and out of the head zone 36, seat zone 38, thigh zone 40, and foot zone 42 to control the interface pressure experienced by the patient supported on the mattress 18. However, the present disclosure is directed to using the information from sensor assembly 60 to make further inferences regarding motion by the patient supported on the mattress 18. The sensor assembly 60 includes separate sensors for measuring the air pressure in each of the head zone 36, seat zone 38, thigh zone 40, and foot zone 42. The pressure sensor assembly includes a head zone sensor 82, a seat zone sensor 84, a thigh zone senor 86, and a foot zone sensor 88. While signals from the sensors 82, 84, 86, and 88 are used to control the pressure in the respective zones, applying the principles of the present disclosure, the signals are also useful in making inferences regarding patient movement and, when used synergistically with the information gleaned from the signals from the load cells 66, 68, 70, and 72, provide a more fulsome and accurate analysis of patient movement and/or motion.

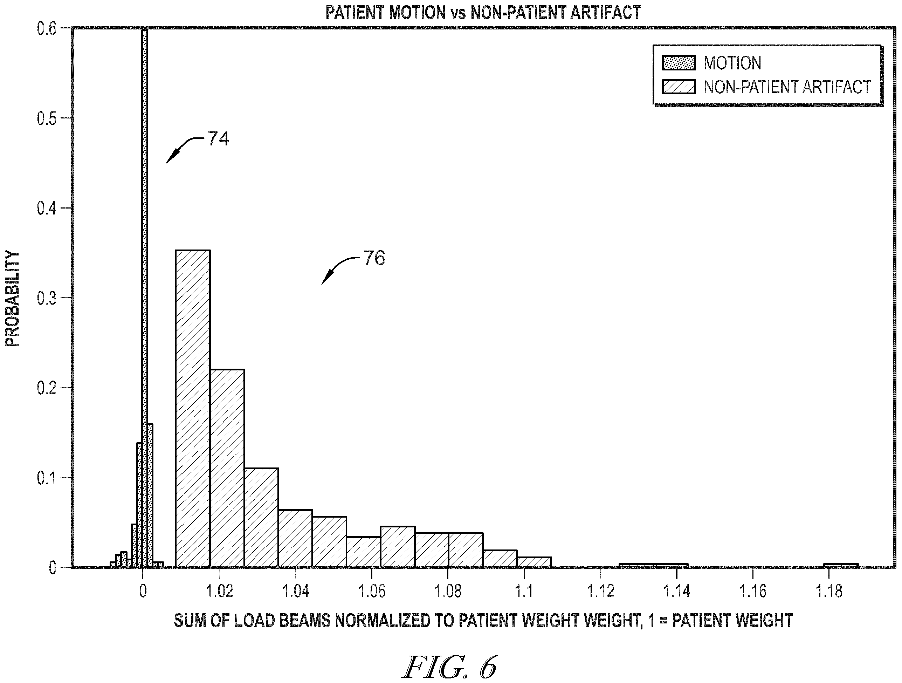

Thus, the present disclosure is directed to utilizing the bed 10, and specifically the scale module 50 and air module 52, as an instrument for measuring the motions of a patient that occupies the bed 10 and characterizing that motion to make inferences about the patient's health. Like all biomedical sensing systems, error can be introduced when the sensor output is affected by various sources of noise. Some sources of noise, such as electrical or stray environmental noise can be mitigated through robust design. However, it is not always possible to mitigate human-caused forces that are imparted to the load cells 66, 68, 70, and 72 that are not generated from solely the patient themselves. These artifacts are referred to herein as non-patient motion artifacts (NPMA). Fig. 6 is a graph demonstrating the relationship between strictly patient motions, shown as narrow columns at reference 74, to NPMAs, shown as wide columns at reference 76. When NPMAs occur, they are typically much greater in motion magnitude than patient motions. Failure to acknowledge and adjust for the presence of these NPMA results in an inaccurate inference of mobility, self-directed motion, etc. to bias towards patients having much more mobility than they actually have.

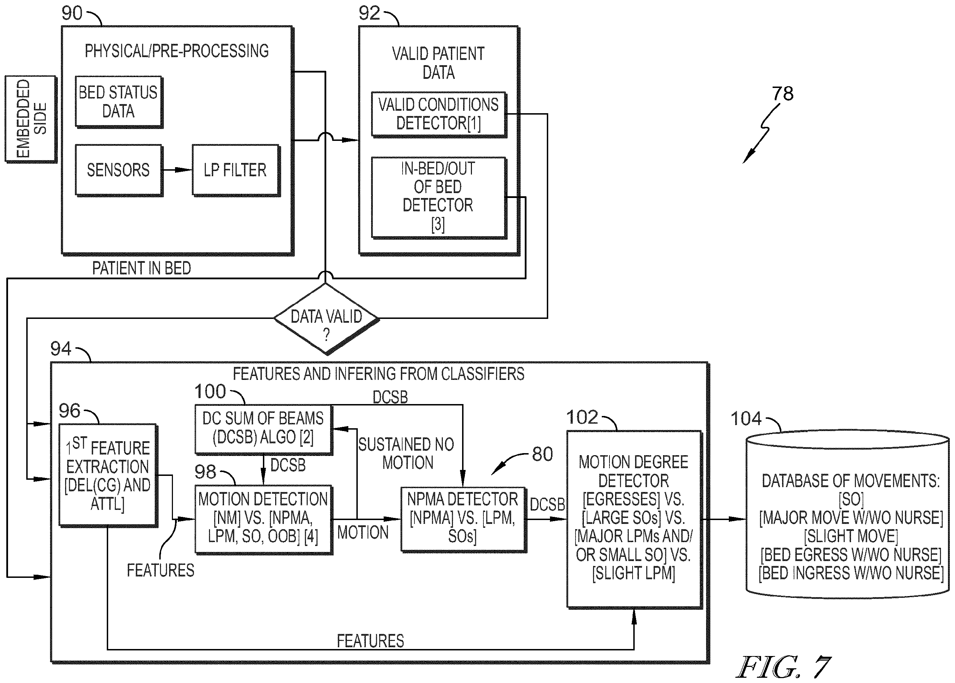

The present disclosure is directed to having a practical and accurate analysis and mitigation of NPMAs in real-time. Once determined, load cell signal data that has aspects of NPMA movement can be mitigated or ignored such that true-patient motion can be determined. Referring now, to Fig. 7, an algorithm 78 for true motion detection is illustrated, including the NPMA detector function 80.

Through an empirical study that included real-time data collection from video observation of test subject patients synchronized with signals from load cells of the scale module of the bed supporting the test subject patients, the types of motion from the where classified in one of three types: lateral patient motions (LPMs); vertical or self-offloading patient movements (SOs); or non-patient motion artifacts (NPMAs). There were also observations that found that load cell signals varied when there was no patient movement. These artifacts were designated as non-movements (NMs). Permutations of these categories, called "complex movements", also including further categorization into combinations including different directionality of the simple movements was also established.

The use of the signals from the load cells of a bed, such as load cells 66, 68, 70, and 72 to determine the equivalent centroid of vertical load supported by the load cells 66, 68, 70, and 72 is known. This centroid/center-of-gravity (CG) approach is used to infer some patient motion. Through empirical analysis, a determination of motion in the x/y plane (see Fig. 1) of a range of speeds and magnitudes of motion that are associated with patient motions has been determined. Thus, this allows for the detection of lateral patient motions (LPMs), which are, by definition, detected patient motions which have no vertical component. Any lateral movement will cause the center of gravity of the bed to change during a unit-time interval, proportionally to ratio of the displacement of the amount of mass moved to the amount of mass that remained stationary. Due to this, this feature is self-normalized by patient weight. Note that CG movement in the x and y axis is merged by typical vector addition and the directionality is ignored to establish a factor called CGspeed. It is understood that both patient and non-patient movements will cause the CG to move, for these reasons, and the classifiers and inference models discussed below, any motion that imparts its force to the weigh frame 30 is considered to be a motion.

In a first approach at discriminating NPMAs from LPMs, the patient and the bed 10 are treated as a closed system. The total energy of the closed system will be constant and conserved over time of typical patient movements. Any energy that is created by the patient as a result of them moving does not change the overall loading of all four load cells 66, 68, 70, and 72, but simply changes the proportion the total load that each load cell 66, 68, 70, or 72 is carrying at any given time. There is no total gain of loads, the loads simply shift around the four load cells 66, 68, 70, and 72 as the patient moves laterally.

In contrast, when a caregiver pushes or pulls on the patient or bed 10 (a NPMA), the closed-system is corrupted by an external energy source and the net load on the load cells 66, 68, 70, and 72 increased or decreased. This is the case for both transient touches of the bed 10, such as when a person hugs the patient, and in sustained touches of the bed 10, such as when a caregiver leans on bed 10 while doing long procedure. In either case, an additional load is introduced to the load cells 66, 68, 70, and 72 resulting in a material change from the sum of the loads on each load cell 66, 68, 70, and 72 when the transient load is applied to the bed 10. The value of the transient load, designated as total transient load (TTL) is calculated by subtracting the from the total load measured by the load cells 66, 68, 70, and 72 the closed-system load measured before the transient event; the closed-system load which is effectively the patient's static weight, designated as the DC sum of beams (DCSB) which can be determined using known techniques, such as that disclosed in U.S. Pat. No. 10,054,479 titled "BED WITH AUTOMATIC WEIGHT OFFSET DETECTION AND MODIFICATION," which is disclosure of monitoring and updating a patient load to establish a static patient weight, DCSB.

Once the DCSB is established, a simple threshold can be tested to determine whether a TTL is a NPMA or not. The units here are forces, measured in kg, also called kg-force. As part of the test of the threshold, an oscillation in the location of the CG and an effective return of the TTL to zero can be considered to confirm the transient nature of the load to help confirm that the event is a TTL.

However, there is an exception to this simple approach. Relying just on thresholding TTL moment-by-moment is confounded by self-offloading patient movements (SOs). SOs are large vertical shifts that are an artifact of a patient quickly lifting their core body up using the strength in his legs or arms and then returning to a starting or near starting position. These self-movements cause large momentary changes in TTL and may appear to be an NPMA. Although SOs can cause momentary large shifts from the patient's weight in the closed system, appearing to break it, the closed system is not broken if the response of the system through the entire duration of the motion is considered.

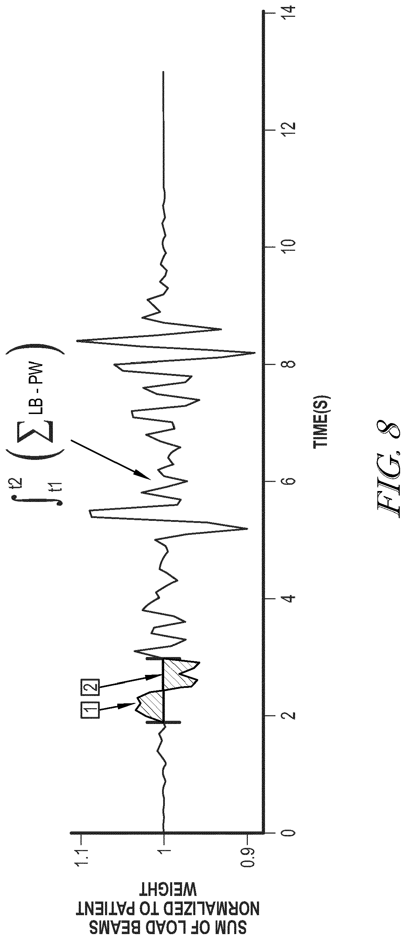

Referring to Fig. 8, the phenomenon of an SO event is illustrated with reference to the variation the sum of the signals from the load cells 66, 68, 70, and 72 over time. The initial forces imparted to move the trunk of the patient create a spike in the measured load as the movement begins to occur in a preload phase designated as 1 on the graph in Fig. 8. The forces oscillate after the movement occurs in a reaction phase, designated as 2 on the graph in Fig. 8. This is a result of a sort of conservation of energy. The integral or sum of TTL over this event is approximately zero when an SO event is experience, where TTL is negative at points in time, such as the designation 2. Under this conservation of energy approach, the SO is detected when any loads induced by the motion of the patient are offset by reactionary loads.

To establish characterization criteria, an empirical study using the synchronized video and load cell signal capture approach discussed above was implemented to gather a mixture of human-generated and human-surrogate test movements were performed. Taking care to vary parameters such as motion speed and magnitude of both patient and NPMA movements, and introducing variability such as different body shapes and strengths in the case of the human subjects, a representative data sample was developed. In the case of the human subjects, three subjects were used a ten-year-old male, 44.5 kg, a fourteen-year-old female, 60 kg, and a forty-year-old male, 101 kg.

Using the empirically generated test data, an approach was developed to model the absolute total transient load (ATTL), which is defined as:

$$ATTL = |\ [\Sigma(RHLC, LHLC, RFLC, LFLC) - DCSB]\ /\ DCSB\ |\quad (EQ.\ 1)$$

Where RHLC, LHLC, RFLC, and LFLC are the values in kg, of the four load cells 66, 68, 70, and 72 and DCSB, which is defined above. This approach provides an absolute value of the TTL, recognizing that transient loads may also unload the weigh frame 30 in some situations.

The conservation of energy theory was modeled using an integral approach and taking the absolute value of the integral as shown below.

$$centInt = \left| \int_L^{-L} [(sum(RHLC, LHLC, RFLC, LFLC) - DCSB)/DCSB] \right| \quad (EQ.\ 2)$$

A third modeling equation was derived to calculate CGspeed, which is discussed in theory above, and these equations were used:

$$CGspeed = \frac{\left(\sqrt{\Delta CGx^2 + \Delta CGy^2}\right)}{t} \quad (EQ.\ 3)$$

$$CGx = X * (LHLC + LFLC)/sum(RHLC, LHLC, RFLC, LFLC) \quad (EQ.\ 4)$$

$$CGy = Y * (RHLC + RFLC)/sum(RHLC, LHLC, RFLC, LFLC) \quad (EQ.\ 5)$$

Where t, is the time interval over which the change in the position of the CG moves and where X is the distance between the left load cells 68, 72 and the right load cells 66, 70, and Y is the distance between head load cells 66, 68 and the foot load cells 70, 72.

The empirically collected test data was manually annotated as patient movement (SO, LPM, or out of bed (OOB)), non-patient motion artifact (NPMA) or no-movement (NM). Patient movements occurring simultaneously with NPMAs were considered labeled as NPMAs, since ultimately it is more important to correctly identify NPMAs when they occur, potentially during patient movement, and throw out all of the data so as not to bias patient movements. To guard against human error of labeling, movements for training and validation were "moved inward", starting 0.5 seconds later and 0.5 before the end as was labeled by humans on all classes. If the (non)movement was greater than 5 seconds, 1 second was used instead of 0.5 seconds. Each feature was calculated in 100 ms intervals, which was determined to be appropriate.

Table 1 below summarizes the different classes used, the expected responses of the features, and the labeling of the response variables used for training for machine learning. As seen in the table, the features discussed differentiate between the different categories and thus a classifier can be built with them.

Table 1

| Simple/ complex | Type of motion | Motion Class | NPMA Class | Predictor ATTL | Predictor centInt | Predictor CGspeed |
|---|---|---|---|---|---|---|
| - | Nomotion (NM) | 0 | 0 | low | low | low |
| simple | LPM | 1 | 0 | low | low | high |
| simple | SO | 1 | 0 | high | high | high |
| simple | NPMA | 1 | 1 | high | low | high |
| complex | LPM_OOB | 1 | 0 | varies | varies | high |
| complex | LPM_ NPMA | 1 | 1 | varies | varies | high |

Data was visualized to confirm the theoretical behavior suspected, as laid out in Table 1. Although it appears that patient motion is predicted by one variable, and NPMA/non-NPMA is determined by two, an approach that would serialize classifiers by determining if there is ANY motion (patient or non-patient) and pass that data into the classifier that determines the presence of NPMA vs. non NPMA was implemented.

A binary logistic regression was used to determine a simple threshold that could easily be deployed in an embedded system. A 10-times K-folds cross-validation with an 80/20 ratio split of the training from the validation data was performed. An AUC-ROC curve was prepared to score the performance of the motion classifier classification approach applied at various thresholds for TPR vs. FPR. The validation test resulted in an ROC AUC of 0.99, which is a near-perfect classifier. For this motion detection model, using a threshold of 0.5, 94% of the time true motion is detected and 99% of the time true no motion is detected.

The coefficients from the empirical model describes the size and direction of the relationship between a predictor feature and the response variables. Using the dataset, the estimated coefficient for the center of gravity speed is -24.43 and change in absolute changes in transient load (ATTL) is -14.57. The intercept of this model is 4.98. Using these coefficients, the following relationship describes motion or no motion:

$$\ln \frac{Pm}{(1-Pm)} = \beta_1 + x_2\beta_2 + x_1\beta_1 + = 14.98 - 24.43 * CGspeed - 14.57 * ATTL \quad (EQ. 6)$$

Where Pm is the probability there is a motion.

Due to the binary shape of a sigmoid (shape that is used in logistic regression), this equation represents a line in a two-dimensional feature space, where it can be said that any data point above this line will be motion and any data point below this line will be no-motion group.

The data was further analyzed with a single factor to test for NPMA events and an estimated threshold coefficient for *centInt* was 2.87 and the intercept was -4.95. The following equation represents a vertical line indicating that any data point to the right side will be NPMA.

$$\ln \frac{Pn}{(1-Pn)} = \beta_0 + x_1\beta_1 = -4.95 + 2.87 * (intCent) \quad (EQ. 7)$$

Where Pn is the probability there is a NPMA event.

Having successfully established that the features under study could be extracted and applied with confidence in an inference model, a generalized algorithm 78 for processing sensor data from existing sensors from a bed 10 was developed as shown in Fig. 7. At a preprocessing step 90, bed status data and sensor data are pre-processed with sensor data being filtered, such as through low-pass filter. Additional testing is confirmed at step 92 where data being transferred into an inference engine 94 is validated. At step 92, validation include a determination that sensor data being received is consistent with the environment of the bed and patient. In some examples, information may be received from a hospital information system 32 which indicates an expected sensor signal range. For example, validation may test the DCSB against the weight of the patient in the hospital information system 32 to validate that the signals from the load cells 66, 68, 70, 72 are reasonable. The hospital information system 32 may include an admission/discharge/transfer (ADT) management system, an electronic medical records system, or a nurse call system. Each of these units of the hospital information system 32 may regularly communicate with others of the systems or may be standalone systems. The validation step 92 may also use other sensors to confirm that a patient is in the bed 10 and generating meaningful data to confirm the validity of the algorithm 78 in real time.

The filtered data is provided to the inference engine 94 where at step 96 a first feature extraction is conducted. From the example above, a first feature is extracted to confirm whether there is a threshold state, such as motion or no motion. The CGspeed and ATTL analysis were each proven to be a useful to establish the presence of motion or no motion. In other embodiments, other first features may extracted as the first step in a serial classification approach. Upon extraction of the first feature, the serial classification approach is continued with the features extracted in step 96 advanced to step 98 where baseline data is tested against an extracted first feature to determine whether a threshold has been met that is indicative of motion. If no motion is detected, the algorithm 78 loops at step 98 until motion is identified and classification can be conducted.

Once motion is identified at step 98, the motion is discriminated at step 80 between NPMA and patient motion of either LPM or SO. If NPMA is identified at step 80, then the signal data is disregarded. However, confirmation and characterization of LPM or SO at step 80 is further analyzed at step 102 to establish a degree of motion. At step 102, in the illustrative embodiment, the motion can be distinguished between an egress, a large self-offloading patient movement (SO), a major lateral patient motion (LPM) and/or a small self-offloading patient movement (SO), or a slight lateral patient motion. Once the inference as to the type of patient motion is complete at step 102, the information is then moved to a database associated with the patient as step 104. For example, at step 104 the patient's medical record can be updated, based on the inference identify, objectively, the patient's motion and behavior such as regular self-offloading patient movement (SO), major lateral patient motions (LPMs), slight LPMs, or ingress or egress with or without caregiver assistance.

In addition to simple characterization of the patient motion, between an LPM, SO, egress, or ingress, the data from the load cells 66, 68, 70, 72 may be processed further to provide a higher level of sensitivity to the characterization of the patient movement. While the discussion above addresses the inference and first characterization of the patient motion, by calculating the work done by the patient during the motion, a more fulsome understanding the magnitude of the patient motion can be used to monitor the patient and provide insights as to the patient's medical progression, whether it be positive or negative.

While the algorithm 78 of Fig. 7 has been established as a successful approach to drawing inferences regarding the characterization of patient motion, considering the entire structure of the bed 10, additional information is available that may be fused with the load cell signal data to provide and even more accurate indication of the status of the patient and assist in improving the inferences drawn. Specifically, the patient is supported on the mattress 18 and portions of the patient are supported on the inflated zones 36, 38, 40, and 42. The respective head zone sensor 82, a seat zone sensor 84, a thigh zone senor 86, and a foot zone sensor 88 are each gathering data in real time and the air module 52 is in communication with the scale module 50 and controller 28 so that data can be shared to further inform the analysis.

Referring to Fig. 4, a diagrammatic side view of a patient supported on the mattress 18 and frame 34 with the load of the patient being borne by the inflated zones 36, 38, 40, and 42 and passed through the mattress to the frame 34 to the load cells 66, 68, 70, and 72 supported from the weigh frame 30. As seen in Fig. 4, in a static condition, the patient's weight is appropriately distributed over the inflated zones 36, 38, 40, and 42. Each of those zones 36, 38, 40, and 42 are inflated to a target pressure based on the patient's weight detected by the scale module 50 and the expected distribution of the patient.

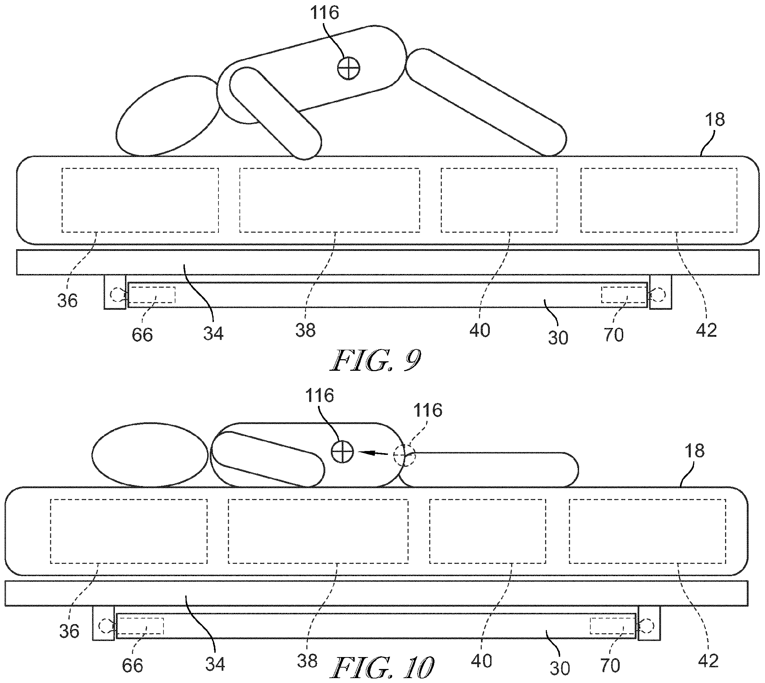

Fig. 9 is similar to Fig. 4, but shows the patient performing a self-offloading patient movement, such as to adjust their position on the bed 10. Rather than being appropriately distributed, in the example of Fig. 9, the patient has drawn their feet up to be positioned over the thigh zone 40 rather than the foot zone 42. Similarly, they have arched their back so that there is a lighter load on the seat zone 38 and an increased load on the head zone 36. Thus, in the instant shown in Fig. 9, the head zone 36 and thigh zone 40 are abnormally heavily loaded and the seat zone 38 and foot zone 42 are abnormally unloaded.

This is the type of offloading that would be identified as consistent with the oscillations of the weigh in Fig. 8 and would likely be appropriately identified as an SO event by the algorithm 78, but supplementing the algorithm with the variations in pressure, and especially the variations in the impulse pressure in zones 36 and 40 are consistent with a patient offloading and inconsistent with a caregiver or visitor contacting the bed 10 or the patient as the external influence is likely to be a concentrated load or unload over a particular zone 36, 38, 40, or 42 or adjacent zones 36, 38, 40, and 42.

Turning now to Fig. 10, it can be seen that the patient has settled into a steady state position, but the patient's center of gravity 116 has moved from the original location shown in Fig. 4 (and indicated in phantom in Fig. 10) to a new location relative to the load cells 66, 68, 70, 72. Thus, the confidence that the detected motion resulted in actual movement is augmented by considering the after event weight distribution, but also by considering the combined readings of the head zone sensor 82, a seat zone sensor 84, a thigh zone senor 86, and a foot zone sensor 88 in cooperation with the load cells 66, 68, 70, and 72, provides a high confidence in the inferences drawn.

Still further, it should be understood the closed-system conservation of energy principles discussed above with regard to the load cells 66, 68, 70, and 72 hold with regard to the collective air pressure sensors 82, 84, 86, 88 and the load cells 66, 68, 70, 72 all-together. In other words, changes in energy at one inflatable zone 36, 38, 40, or 42 should be conserved, but may not be conserved as measure only by the air pressure sensors 82, 84, 86, 88, but some of the energy may be transferred away from the sensors 82, 84, 86, 88 and measured at one or more of the load cells 66, 68, 70, 72. Similarly, energy transferred away from one of the load cells 66, 68, 70, 72 and be effectively measured by one or more of the air pressure sensors 82, 84, 86, 88. This is best understood with an appreciation for the relative flexibility of the inflatable zones 36, 38, 40, or 42 and mattress 18 in total. The ability of the mattress 18 to absorb "shocks" without transferring the concomitant energy change to the load cells 66, 68, 70, 72 by transferring the energy to stretching fabric or compressing air within the zones 36, 38, 40, 42 confounds the analysis.

However, following the basic principles of conservation of energy, for movements considered to be NPMAs, a simple test to monitor for net changes in energy in the entire system of sensors will provide a high confidence level that the detected movement is, or is not, a true patient movement. In effect, the net kinetic energy being measured by the air pressure sensors 82, 84, 86, 88 and load cells 66, 68, 70, 72 should stay relatively constant, other than the theoretical variations due to heat transfer.

Finally, the implementation requires the controller 28 to account for energy added to the system by the operation of components of the bed 10 such as the blower 106 or various drive motors that move components of the frame 34, such as the head section 44.

With this in mind, we return to the control system 26 shown in Fig. 2. The control system 26 further includes a communications interface 108 that is operable, under the control of the controller 28, to communicate with the hospital information system 32 through a communications infrastructure 110 to share the patient health characterization, whether that be a mobility score, an activity score, a consciousness score, or any other objective score based on the output from the bed 10 acting as a sensor to objectively measure the motions made by the patient and characterizing the type of motions patient is making.

Still further, it is contemplated that if the controller 28 detects an adverse condition, the controller 28 may communicate that adverse condition through the communications interface 108 to the hospital information system 32 for action by caregivers. Similarly, the controller 28 may communicate an adverse event to the user interface 54 which may issue an audible or visual alert of the adverse condition. The adverse condition may be based on an acceptable threshold of motion or work. In addition, the adverse condition evaluation may rely solely on a rate of change of patient motion or work. For example, a significant drop in the motion of or work being done by a patient may be an indicator of the deterioration of a patient due to, for example, sepsis, delirium, or a loss of consciousness.

## Claims

1. A system comprising

   a patient support surface (10) including a plurality of inflatable zones (36, 38, 40, 42),
   a plurality of load cells (66, 68, 70, 72) supporting the patient support surface,
   a plurality of air pressure sensors (82, 84, 86, 88), each pressure sensor operable to measure the pressure in a respective inflatable zone of the patient support surface, and
   a controller (28) operable to receive a separate signal from each of the plurality of load cells and each of the plurality of air pressure sensors, process the signals to identify motion of the patient, the signals being further processed to distinguish the type of patient motion between a self-offloading motion and a lateral patient motion and, based on the type of patient motion, automatically update a patient profile in a patient record to reflect the type of patient motion.

2. The system of claim 1, wherein the controller is operable to monitor the energy detected by each of the load cells and each of the air pressure sensors and compare the change in total energy measured by the load cells and air pressure sensors to determine if external energy has acted on the system, and, if a change in total energy measured is indicative that external energy has acted on the system, utilize the resulting change in total energy to modify the type of patient motion that is used to update the patient profile.

3. The system of either claim 1 or claim 2, wherein the signals are indicative of motion of a least a portion of the patient in a vertical direction.

4. The system of any preceding claim, wherein the controller is operable to calculate the work done by the patient in the vertical direction to distinguish the patient motion.

5. The system of any preceding claim, wherein the controller is operable to compare the air pressure sensor signal for each of two adjacent zones and use the relative changes in the pressure in the adjacent zones during a potential patient movement event to confirm the type of patient motion.

6. The system of claim 5, wherein the controller is operable to monitor the energy detected by each of the load cells and each of the air pressure sensors and compare the change in total energy measured by the load cells and air pressure sensors to determine if external energy has acted on the system, and, if a change in total energy measured is indicative that external energy has acted on the system, utilize the resulting change in total energy to modify the type of patient motion that is used to update the patient profile.

7. The system of either claim 5 or claim 6, wherein the potential patient motion is distinguished as a self-offloading motion in which a patient readjusts their position on the patient support surface without any external influence.

8. The system of any one of claims 5 to 7, wherein the potential patient motion is distinguished as a non-patient movement artifact in which a patient's position is changed on the patient support surface with external influence.

9. The system of any one of claims 5 to 8, wherein the potential patient motion is distinguished as a patient lateral motion in which a patient readjusts their position on the patient support surface without any external influence.

10. The system of any preceding claim, wherein the controller is operable to determine that the distinguished patient motion results in an adverse condition for the patient and issues an alert indicative of the adverse condition.

11. The system of any preceding claim wherein the patient support comprises a frame (20) supported on the load cells, a mattress (18) including the plurality of inflatable zones positioned on the frame, the mattress and frame cooperating to direct any patient load through the mattress and frame to the load cells, and wherein the controller is operable to process the signals to identify, based on transient changes in the signals, motion of the patient that does not result in relative movement of the patient relative to the frame.

12. A method of distinguishing motion of a person on an inflatable mattress (18) having multiple inflatable zones (36, 38, 40, 42), by using the system of claim 1, the method comprising the steps of:

    monitoring signals from a plurality of pressure sensors (82, 84, 86, 88), each pressure sensor operable to provide a signal indicative of the pressure in a respective inflatable zone;
    monitoring signals from a plurality of load cells (66, 68, 70, 72), the plurality of load cells supporting the inflatable mattress;
    processing the signals from the load cells and the pressure sensors to identify motion of the person;
    upon detection of a potential motion of the person, further processing the signals to distinguish the type of person's motion between a self-offloading motion and a lateral patient motion; and
    based on the type of motion, automatically updating a record associated with the particular person to reflect the type of motion of the person.

13. The method of claim 12, further comprising:

    monitoring the energy detected by each of the load cells and each of the air pressure sensors;
    comparing the change in total energy measured by the load cells and air pressure sensors to determine if external energy other than the person supported on the mattress has acted on the mattress; and,
    if a change in total energy measured is indicative that external energy has acted on the mattress, utilizing the resulting change in total energy to modify the type of motion that is used to update the record.

14. The method of either claim 12 or claim 13, further comprising:

comparing the air pressure sensor signal for each of two adjacent zones; and
using the relative changes in the pressure in the adjacent zones during a potential person movement event to confirm the type of motion.

**15.** The method of claim 14, further comprising:

monitoring the energy detected by each of the load cells and each of the air pressure sensors;
comparing the change in total energy measured by the load cells and air pressure sensors to determine if external energy has acted on the mattress; and,
if a change in total energy measured is indicative that external energy has acted on the mattress, utilizing the resulting change in total energy to modify the type of motion that is used to update the record.

**Patentansprüche**

**1.** System, das Folgendes umfasst:

eine Patientenauflagefläche (10) mit mehreren aufblasbaren Zonen (36, 38, 40),
mehrere die Patientenauflagefläche tragende Kraftmessdosen (66, 68, 70, 72),
mehrere Luftdrucksensoren (82, 84, 86, 88), wobei jeder Drucksensor die Aufgabe hat, den Druck in einer jeweiligen aufblasbaren Zone der Patientenauflagefläche zu messen, und
eine Steuerung (28) mit der Aufgabe, ein separates Signal von jeder der mehreren Kraftmessdosen und jedem der mehreren Luftdrucksensoren zu empfangen, die Signale zu verarbeiten, um Bewegung des Patienten zu identifizieren, wobei die Signale weiter verarbeitet werden, um die Art der Patientenbewegung zwischen einer Selbstentlastungsbewegung und einer lateralen Patientenbewegung zu unterscheiden und, auf der Basis der Art der Patientenbewegung, automatisch ein Patientenprofil in einer Patientendatei zu aktualisieren, um die Art der Patientenbewegung zu reflektieren.

**2.** System nach Anspruch 1, wobei die Steuerung die Aufgabe hat, die von jeder der Kraftmessdosen und jedem der Luftdrucksensoren erfasste Energie zu überwachen und die Änderung der von den Kraftmessdosen und Luftdrucksensoren gemessenen Gesamtenergie zu vergleichen, um festzustellen, ob externe Energie auf das System eingewirkt hat, und, wenn eine Änderung der gemessenen Gesamtenergie anzeigt, dass externe Energie auf das System eingewirkt hat, die resultierende Änderung der Gesamtenergie zu nutzen, um die zur Aktualisierung des Patientenprofils verwendete Art der Patientenbewegung zu modifizieren.

**3.** System nach Anspruch 1 oder Anspruch 2, wobei die Signale Bewegung zumindest eines Teils des Patienten in vertikaler Richtung anzeigen.

**4.** System nach einem vorherigen Anspruch, wobei die Steuerung die Aufgabe hat, die vom Patienten in vertikaler Richtung geleistete Arbeit zu berechnen, um die Patientenbewegung zu unterscheiden.

**5.** System nach einem vorherigen Anspruch, wobei die Steuerung die Aufgabe hat, das Luftdrucksensorsignal für jede von zwei benachbarten Zonen zu vergleichen und die relativen Änderungen des Drucks in den benachbarten Zonen während eines potenziellen Patientenbewegungsereignisses zum Bestätigen der Art der Patientenbewegung zu nutzen.

**6.** System nach Anspruch 5, wobei die Steuerung die Aufgabe hat, die von jeder der Kraftmessdosen und jedem der Luftdrucksensoren erfasste Energie zu überwachen und die Änderung der von den Kraftmessdosen und Luftdrucksensoren gemessenen Gesamtenergie zu vergleichen, um festzustellen, ob externe Energie auf das System eingewirkt hat, und, wenn eine Änderung der gemessenen Gesamtenergie anzeigt, dass externe Energie auf das System eingewirkt hat, die resultierende Änderung der Gesamtenergie zum Modifizieren der zur Aktualisierung des Patientenprofils verwendeten Art der Patientenbewegung zu nutzen.

**7.** System nach Anspruch 5 oder Anspruch 6, wobei die potenzielle Patientenbewegung als eine Selbstentlastungsbewegung unterschieden wird, bei der ein Patient seine Position auf der Patientenauflagefläche ohne jeglichen externen Einfluss neu einstellt.

**8.** System nach einem der Ansprüche 5 bis 7, wobei die potenzielle Patientenbewegung als Nicht-Patienten-Bewegungsartefakt unterschieden wird, bei dem die Position eines Patienten auf der Patientenauflagefläche mit externem Einfluss verändert wird.

9. System nach einem der Ansprüche 5 bis 8, wobei die potenzielle Patientenbewegung als eine laterale Patientenbewegung unterschieden wird, bei der ein Patient seine Position auf der Patientenauflagefläche ohne externen Einfluss neu einstellt.

10. System nach einem vorherigen Anspruch, wobei die Steuerung die Aufgabe hat festzustellen, dass die unterschiedene Patientenbewegung zu einem ungünstigen Zustand für den Patienten führt, und einen Alarm ausgibt, der den ungünstigen Zustand anzeigt.

11. System nach einem vorherigen Anspruch, wobei die Patientenauflage einen auf den Kraftmessdosen getragenen Rahmen (20), eine Matratze (18) mit mehreren auf dem Rahmen positionierten aufblasbaren Zonen umfasst, wobei die Matratze und der Rahmen zusammenwirken, um jegliche Patientenbelastung durch die Matratze und den Rahmen zu den Kraftmessdosen zu leiten, und wobei die Steuerung die Aufgabe hat, die Signale zu verarbeiten, um auf der Basis transienter Änderungen in den Signalen Bewegung des Patienten zu identifizieren, die nicht zu einer relativen Bewegung des Patienten relativ zum Rahmen führt.

12. Verfahren zum Unterscheiden von Bewegung einer Person auf einer aufblasbaren Matratze (18) mit mehreren aufblasbaren Zonen (36, 38, 40, 42) unter Verwendung des Systems nach Anspruch 1, wobei das Verfahren die folgenden Schritte beinhaltet:

Überwachen von Signalen von mehreren Drucksensoren (82, 84, 86, 88), wobei jeder Drucksensor die Aufgabe hat, ein Signal zu liefern, das den Druck in einer jeweiligen aufblasbaren Zone anzeigt;
Überwachen von Signalen von mehreren Kraftmessdosen (66, 68, 70, 72), wobei die mehreren Kraftmessdosen die aufblasbare Matratze tragen;
Verarbeiten der Signale von den Kraftmessdosen und den Drucksensoren, um Bewegung der Person zu erkennen;
weiteres Verarbeiten der Signale bei Erkennung einer potenziellen Bewegung der Person, um die Art der Bewegung der Person zwischen einer Selbstentlastungsbewegung und einer lateralen Patientenbewegung zu unterscheiden; und
automatisches Aktualisieren, auf der Basis der Art der Bewegung, eines mit der bestimmten Person assoziierten Datensatzes, um die Art von Bewegung der Person zu reflektieren.

13. Verfahren nach Anspruch 12, das ferner Folgendes beinhaltet:

Überwachen der von jeder der Kraftmessdosen und jedem der Luftdrucksensoren erfassten Energie;
Vergleichen der Änderung der von den Kraftmessdosen und Luftdrucksensoren gemessenen Gesamtenergie, um festzustellen, ob eine andere externe Energie als die auf der Matratze gelagerte Person auf die Matratze eingewirkt hat; und
Nutzen, wenn eine Änderung der gemessenen Gesamtenergie anzeigt, dass externe Energie auf die Matratze eingewirkt hat, der resultierenden Änderung der Gesamtenergie, um die Art von Bewegung zu modifizieren, die zum Aktualisieren des Datensatzes genutzt wird.

14. Verfahren nach Anspruch 12 oder Anspruch 13, das ferner Folgendes beinhaltet:

Vergleichen des Luftdrucksensorsignals für jede von zwei benachbarten Zonen; und
Nutzen der relativen Änderungen des Drucks in den benachbarten Zonen während eines potenziellen Personenbewegungsereignisses zum Bestätigen der Art von Bewegung.

15. Verfahren nach Anspruch 14, das ferner Folgendes beinhaltet:

Überwachen der von jeder der Kraftmessdosen und jedem der Luftdrucksensoren erfassten Energie,
Vergleichen der Änderung der von den Kraftmessdosen und Luftdrucksensoren gemessenen Gesamtenergie, um festzustellen, ob externe Energie auf die Matratze eingewirkt hat; und
Nutzen, wenn eine Änderung der gemessenen Gesamtenergie anzeigt, dass externe Energie auf die Matratze eingewirkt hat, der resultierenden Änderung der Gesamtenergie, um die zur Aktualisierung des Datensatzes verwendete Art von Bewegung zu modifizieren.

**Revendications**

1. Système comprenant

une surface de support de patient (10) comportant une pluralité de zones gonflables (36, 38, 40, 42),
une pluralité de cellules de charge (66, 68, 70, 72) supportant la surface de support de patient,
une pluralité de capteurs de pression d'air (82, 84, 86, 88), chaque capteur de pression étant exploitable pour mesurer la pression dans une zone gonflable respective de la surface de support de patient, et
un contrôleur (28) exploitable pour recevoir un signal distinct à partir de chacune de la pluralité de cellules de charge et de chacun de la pluralité de capteurs de pression d'air, traiter les signaux pour identifier un mouvement du patient, les signaux étant traités en outre pour différencier le type de mouvement de patient entre un mouvement d'auto-délestage et un mouvement de patient latéral et, en fonction du type de mouvement de patient, mettre automatiquement à jour un profil de patient dans un dossier de patient pour refléter le type de mouvement de patient.

2. Système selon la revendication 1, dans lequel le contrôleur est actionnable pour surveiller l'énergie détectée par chacune des cellules de charge et chacun des capteurs de pression d'air et comparer le changement de l'énergie totale mesurée par les cellules de charge et les capteurs de pression d'air afin de déterminer qu'une énergie externe a agi ou non sur le système, et, si un changement de l'énergie totale mesurée indique qu'une énergie externe a agi sur le système, utiliser le changement résultant de l'énergie totale pour modifier le type de mouvement de patient qui est utilisé pour mettre à jour le profil de patient.

3. Système selon la revendication 1 ou la revendication 2, dans lequel les signaux sont indicatifs d'un mouvement d'au moins une partie du patient dans une direction verticale.

4. Système selon l'une quelconque des revendications précédentes, dans lequel le contrôleur est exploitable pour calculer le travail effectué par le patient dans la direction verticale afin de différencier le mouvement de patient.

5. Système selon l'une quelconque des revendications précédentes, dans lequel le contrôleur est exploitable pour comparer le signal de capteur de pression d'air pour chacune de deux zones adjacentes et utiliser les changements relatifs de la pression dans les zones adjacentes durant un événement potentiel de mouvement de patient pour confirmer le type de mouvement de patient.

6. Système selon la revendication 5, dans lequel le contrôleur est exploitable pour surveiller l'énergie détectée par chacune des cellules de charge et chacun des capteurs de pression d'air et comparer le changement de l'énergie totale mesurée par les cellules de charge et les capteurs de pression d'air pour déterminer qu'une énergie externe a agi ou non sur le système, et, si un changement de l'énergie totale mesurée indique qu'une énergie externe a agi sur le système, utiliser le changement résultant de l'énergie totale pour modifier le type de mouvement de patient qui est utilisé pour mettre à jour le profil de patient.

7. Système selon la revendication 5 ou la revendication 6, dans lequel le mouvement de patient potentiel est différencié en tant que mouvement d'auto-délestage dans lequel un patient réajuste sa position sur la surface de support de patient sans aucune influence extérieure.

8. Système selon l'une quelconque des revendications 5 à 7, dans lequel le mouvement de patient potentiel est différencié en tant qu'artefact de mouvement non de patient dans lequel la position d'un patient est modifiée sur la surface de support de patient avec une influence externe.

9. Système selon l'une quelconque des revendications 5 à 8, dans lequel le mouvement de patient potentiel est différencié en tant que mouvement de patient latéral dans lequel un patient réajuste sa position sur la surface de support de patient sans aucune influence externe.

10. Système selon l'une quelconque des revendications précédentes, dans lequel le contrôleur est exploitable pour déterminer que le mouvement de patient différencié entraîne une condition défavorable pour le patient et émet une alerte indicative de la condition défavorable.

11. Système selon l'une quelconque des revendications précédentes, dans lequel le support de patient comprend un cadre (20) supporté sur les cellules de charge, un matelas (18) comportant la pluralité de zones gonflables positionnées sur le cadre, le matelas et le cadre coopérant pour diriger toute charge de patient à travers le matelas et le cadre vers les cellules de charge, et dans lequel le contrôleur est exploitable pour traiter les signaux afin d'identifier, sur la base de changements transitoires des signaux, un mouvement du patient qui n'entraîne pas de mouvement relatif du patient par rapport au cadre.

12. Procédé de distinction d'un mouvement d'une personne sur un matelas gonflable (18) présentant de multiples zones gonflables (36, 38, 40, 42), à l'aide du système selon la revendication 1, le procédé comprenant les étapes suivantes :

la surveillance de signaux en provenance d'une pluralité de capteurs de pression (82, 84, 86, 88), chaque capteur de pression étant exploitable pour fournir un signal indicatif de la pression dans une zone gonflable respective ;

la surveillance de signaux en provenance d'une pluralité de cellules de charge (66, 68, 70, 72), la pluralité de cellules de charge supportant le matelas gonflable ;

le traitement des signaux en provenance des cellules de charge et des capteurs de pression pour identifier un mouvement de la personne ;

à la détection d'un mouvement potentiel de la personne, le traitement additionnel des signaux pour différencier le type de mouvement de la personne entre un mouvement d'auto-délestage et un mouvement de patient latéral ; et

en fonction du type de mouvement, la mise à jour automatique d'un dossier associé à la personne en question pour refléter le type de mouvement de la personne.

13. Procédé selon la revendication 12, comprenant en outre :

la surveillance de l'énergie détectée par chacune des cellules de charge et chacun des capteurs de pression d'air ;

la comparaison du changement de l'énergie totale mesurée par les cellules de charge et les capteurs de pression d'air pour déterminer qu'une énergie externe autre que celle de la personne supportée sur le matelas a agi ou non sur le matelas ; et

si un changement de l'énergie totale mesurée indique qu'une énergie externe a agi sur le matelas, l'utilisation du changement résultant de l'énergie totale pour modifier le type de mouvement utilisé pour mettre à jour le dossier.

14. Procédé selon la revendication 12 ou la revendication 13, comprenant en outre :

la comparaison du signal de capteur de pression d'air pour chacune de deux zones adjacentes ; et

l'utilisation des changements relatifs de la pression dans les zones adjacentes durant un mouvement de personne potentiel pour confirmer le type de mouvement.

15. Procédé selon la revendication 14, comprenant en outre :

la surveillance de l'énergie détectée par chacune des cellules de charge et chacun des capteurs de pression d'air ;

la comparaison du changement de l'énergie totale mesurée par les cellules de charge et les capteurs de pression d'air pour déterminer qu'une énergie externe a agi ou non sur le matelas ; et

si un changement de l'énergie totale mesurée est indicatif qu'une énergie externe a agi sur le matelas, l'utilisation du changement résultant de l'énergie totale pour modifier le type de mouvement utilisé pour mettre à jour le dossier.

FIG. 1

FIG. 2

*FIG. 3*

*FIG. 4*

46

36

HEAD ZONE

SEAT ZONE

38

THIGH ZONE

46

FOOT ZONE

42

48

18

*FIG. 5*

PATIENT MOTION vs NON-PATIENT ARTIFACT

MOTION
NON-PATIENT ARTIFACT

PROBABILITY

SUM OF LOAD BEAMS NORMALIZED TO PATIENT WEIGHT WEIGHT, 1 = PATIENT WEIGHT

*FIG. 6*

FIG. 7

$$\int_{t1}^{t2} \left( \sum_{} LB - PW \right)$$

FIG. 8

FIG. 9

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200297564 A1
- US 10054479 B